# EUROPEAN PATENT SPECIFICATION

(11) **EP 2 217 201 B1**
(45) Date of publication and mention of the grant of the patent: **19.12.2018**
(21) Application number: 08848847.3
(22) Date of filing: 30.10.2008
(51) Int. Cl.: A61K 8/44, A61K 8/46, A61Q 19/08

(54) **COMPOSITION FOR TOPICAL USE TO ACHIEVE A RAPID AND INTENSE LIFTING EFFECT**
ZUSAMMENSETZUNG ZUR TOPISCHEN ANWENDUNG FÜR SCHNELLEN UND INTENSIVEN STRAFFUNGSEFFEKT
COMPOSITION À USAGE LOCAL PERMETTANT D'OBTENIR UN EFFET DE LIFTING RAPIDE ET INTENSE

(30) Priority: 15.11.2007 IT BS20070177
(43) Date of publication of application: 18.08.2010
(73) Proprietor: Exalya S.r.l., 25010 San Felice del Benaco (Brescia) (IT)
(72) Inventor: Paoli Ambrosi, Gianfranco, 25087 Salò (Brescia) (IT)
(74) Representative: Sangiacomo, Fulvia
(86) International application number: PCT/IT2008/000674
(87) International publication number: WO 2009/063521

(56) References cited:
- DE-A1-102005 049 664
- FR-A- 2 873 024
- US-A1- 2002 022 052
- US-A1- 2004 067 212
- DATABASE WPI Week 200050 Thomson Scientific, London, GB; AN 2000-545731 XP002526477 & JP 2000 186028 A (KOGA N) 4 July 2000 (2000-07-04)
- "Skin external pharmaceutical prepn. for improving flexibility and elasticity - contains taurine and crude drug extracts, e.g. Lamium album extract" DERWENT,, 1 January 1900 (1900-01-01), XP002333552
- DATABASE gnpd [Online] Mintel; February 2007 (2007-02), Mintel: "Renewing mask", Database accession no. 658194

## Description

### Field of the Invention

The object of this invention is a new cosmetic composition designed to be used to obtain an immediate "lifting" effect, lasting between 6 to 8 hours and able to improve the aesthetic characteristics of the cutis, clear to see from how the skin tension increases, from the reduced visibility of the thin wrinkles and the deeper ones, and from the colour, the tonicity and suppleness of the skin.

### State of the art

Arginine, 2-amino-5-(diaminemethylidene amino) a pentanoic acid, CAS 72-79-3, with molecular formula C₆H₁₄N₄O₂, is a basic semi-essential amino acid able to carry out numerous functions in the human organism. Arginine carries out an important function in the metabolism of nitrogen and it is divided by the anginas enzyme into ornithine and urea.

Arginine produces nitric oxide (NO), an important intra- and intercellular messenger able to inhibit the proliferation of keratinocytes by stimulating the differentiation; this effect is due to the concentration, at low doses it behaves as a stimulator of the proliferation of the keratinocytes, whereas when the concentration is high it stops the cellular proliferation promoting the differentiation.

Taurine, acid 2-aminoethanesulfonic acid, CAS 107-35-7, with molecular formula C₂H₇NO₃S, is a conditionally essential amino acid, which principally differs from amino acids because of the presence of the sulfone group (SOOOH) in place of the traditional carboxylic group (COOH).

Taurine is present in millimolar quantities in the intercellular space. It is very soluble in water and is characterized by low lipophilia values. Together with zinc, taurine is also important for sight and health of the eyes.

Taurine is particularly concentrated as regards to the leukocytes, the skeletal muscles, the heart and central nervous system (it regularizes the transmission of the nervous impulses and stabilizes the cell membranes).

Taurine is a kind of chemical intermediary, able to carry out specific functions with regard to keratinocytes. It functions like an osmolite in keratinocytes maintaining the hydration of the same in the superficial layers of the epidermis, that is the most exposed to atmospheric agents such as sun radiation, wind, cold, etc. Taurine protects the proteins of the keratinocytes from denaturizing should hydration of the granular layer be insufficient.

It was surprisingly shown by the inventor that the association between arginine and taurine, opportunely diffused for topical use, is able to carry out an immediate "lifting" effect; more precisely to carry out a perceptible and visible ironing of the cutis, with the result that the visibility of both the fine and deep wrinkles was reduced. The contemporaneous combined use of arginine and taurine is able to increase cutaneous elasticity, glow and more in general the aesthetic quality of the cutis. This effect is transitory and, depending on the concentration used, it can last from a few minutes to several hours.

This invention has been therefore conceived on the basis of these facts and it provides correspondingly a composition that contains as the active ingredients arginine and taurine in combination, for derma cosmetic treatment aimed at achieving a rapid "lifting" effect and at reaching aesthetic results similar to those achieved by means of intradermic injections of botulinum toxin.

Document US 2004/067212 relates to a skin conditioner comprising as active ingredients L-arginine and/or ethanolamine and possibly taurine, which is used for the treatment of skin ageing symptoms.

### Detailed Description of the Invention

In this invention arginine and taurine can be used in different concentrations depending on the effect required and the chemical-physics form used. Arginine can be used in a concentration in w% varying from 0,5% to 5,0%, whereas taurine can be used in quantities w% from 0,5% to 5,0%.

From the tests carried out by the inventor it was found that the association between taurine and arginine is neither cytotoxic (IC50 > 5) nor phototoxic.

Surprisingly the association between arginine and taurine has also shown to possess a marked effect against the formation of free radicals. Because of these characteristics this association is found to be useful also against cutaneous aging damage and, at the same time, in noticeably improving the aesthetic property of the cutis.

### Examples of preparations with arginine and taurine base

**PREPARATION 2 - monophasic gel with lifting effect**

| N° | Description | w% |
|---|---|---|
| 01 | Arginine | 4,5 |
| 02 | Taurine | 1,2 |
| 03 | Ethyl alcohol | 10, |
| 04 | Carbomer | 1,0 |
| 05 | Deionised water a.r. | 100,00 |

Preparation method: dissolve 01 + 02 in 05; add 03 to the solution obtained; disperse 04 in the solution. All the operation is to be carried out while shaking until a monophasic gel is formed.

**PREPARATION 3 - oil in water emulsion**

| N° | Description | w% |
|---|---|---|
| Phase A) | | |
| 01 | Steareth 2 | 3,0 |
| 02 | Steareth 21 | 2,0 |
| 03 | PPG-15 stearyl ether | 8,0 |
| 04 | Butylhydroxytoluene | 0,01 |

| Phase B) | | |
|---|---|---|
| 05 | Arginine | 1,0 |
| 06 | Taurine | 1,5 |
| 07 | Ethyl alcohol | 10,00 |
| 08 | Deionised water | 5, 0 |
| 09 | Propylene glycol | 1, 5 |

| Phase C) | | |
|---|---|---|
| 10 | Phenoxyethanol | 0,8 |
| 11 | Methyl paraben sodium chloride | 0,25 |
| 12 | Water a.r. | 100 |

Preparation method: Phase A) mix 01+02+03+04 and heat up to 75°C; Phase B) dissolve 06 in 07, then dissolve 05, then mix 08; Phase C) dissolve 11 in 12; mix 10+09 in the solution obtained and heat up to 75°C. At the temperature of 75°C add Phase A) to Phase C); leave to shake; at the temperature of 35°C add Phase B).

**PREPARATION 4 - monophasic solution**

| N° | Description | w% |
|---|---|---|
| 01 | Arginine | 4,0 |
| 02 | Taurine | 1,0 |
| 03 | Ethyl alcohol | 15, 0 |
| 04 | Water a.r. | 100 |

Preparation method: dissolve 01 + 02 in 04; mix 03 into the solution obtained.

## Claims

1. Use for a non-therapeutical treatment of chronologic and photo induced cutaneous aging of a composition comprising arginine in a quantity from 0,5% w/w to 5% w/w and taurine in a quantity from 0,5% w/w to 5% w/w.

2. Use according to claim 1, of a composition comprising arginine in a quantity from 0,5% w/w to 5% w/w and taurine in a quantity from 0,5% w/w to 5% w/w, for an immediate and transitory lifting effect of the cutis, lasting from a few minutes to eight hours and improving the aesthetic parameters such as elasticity, compactness and cutaneous luminosity.

## Patentansprüche

1. Verwendung einer für eine nicht-therapeutische Behandlung von chronologischer und photoinduzierter Hautalterung vorgesehenen Zusammensetzung enthaltend Arginin in einer Menge von 0.5 Gew.-% bis 5 Gew.-% und Taurin in einer Menge von 0.5 Gew.-% bis 5 Gew.-%

2. Verwendung nach Anspruch 1 einer Zusammensetzung enthaltend Arginin in einer Menge von 0.5 Gew.-% bis 5 Gew.-% und Taurin in einer Menge von 0.5 Gew.-% bis 5 Gew.-%, für einen sofortigen und transitorischen Lifting-Effekt der Haut, der einige Minuten bis acht Stunden dauert und die ästhetischen Parameter wie Elastizität, Kompaktheit und Hauthelligkeit verbessert.

## Revendications

1. Usage pour un traitement non thérapeutique du vieillissement cutané chronologique et photo-induit d'une composition comprenant de l'arginine en une quantité de 0.5% p/p à 5% p/p et de la taurine en une quantité de 0.5% p/p à 5% p/p.

2. Usage selon la revendication 1, d'une composition comprenant de l'arginine en une quantité de 0.5% p/p à 5% p/p et de la taurine en une quantité de 0.5% p/p à 5% p/p, pour un effet lifting immédiat et transitoire de la peau, ayant une durée de quelques minutes à huit heures et améliorant les paramètres esthétiques tels que l'élasticité, la compacité et la luminosité cutanée.
